# EUROPÄISCHE PATENTSCHRIFT

(11) **EP 1 618 068 B1**
(45) Veröffentlichungstag und Bekanntmachung des Hinweises auf die Patenterteilung: **16.08.2006**
(21) Anmeldenummer: 04727522.7
(22) Anmeldetag: 15.04.2004
(51) Int. Cl.: C01C 3/02, C01C 3/04, C07C 253/00

(54) **VERFAHREN ZUR AUFREINIGUNG VON BLAUSÄURE**
METHOD FOR PURIFYING HYDROCYANIC ACID
PROCEDE DE PURIFICATION D'ACIDE CYANHYDRIQUE

(30) Priorität: 17.04.2003 DE 10317929
(43) Veröffentlichungstag der Anmeldung: 25.01.2006
(73) Patentinhaber: BASF Aktiengesellschaft, 67056 Ludwigshafen (DE)
(72) Erfinder: BARTSCH, Michael, 67433 Neustadt (DE); BAUMANN, Robert, 68159 Mannheim (DE); HADERLEIN, Gerd, 67269 Gründstadt (DE); FLORES, Miguel, 28300 Aranjuez (Madrid) (ES); JUNGKAMP, Tim, 2950 Kapelle (BE); LUYKEN, Hermann, 67069 Ludwigshafen (DE); SCHEIDEL, Jens, 69493 Hirschberg (DE); SIEGEL, Wolfgang, 67117 Limburgerhof (DE); KUNSMANN-KEITEL, Dagmar, Pascale, 67117 Limburgerhof (DE); BASSLER, Peter, 68519 Viernheim (DE)
(74) Vertreter: Isenbruck, Günter
(86) Internationale Anmeldenummer: PCT/EP2004/003973
(87) Internationale Veröffentlichungsnummer: WO 2004/092068

(56) Entgegenhaltungen:
- WO-A-97/45369
- DE-A- 3 334 321
- DE-B- 1 205 064
- FR-A- 1 377 939
- GB-A- 1 396 249
- US-A- 2 571 099

## Beschreibung

Die Erfindung betrifft ein Verfahren zur Aufreinigung von Blausäure durch Destillation sowie ein Verfahren zur Hydrocyanierung von Olefinen oder Dienen.

Blausäure wird großtechnisch nach im Wesentlichen drei verschiedenen Verfahren hergestellt. Nach einem ersten Verfahren wird Blausäure durch Ammoxidation von Methan mit Sauerstoff und Ammoniak (Andrussow-Verfahren) erhalten. Nach einem zweiten Verfahren wird Blausäure aus Methan und Ammoniak durch Ammondehydrierung in Abwesenheit von Sauerstoff erhalten (BMA-Verfahren der Degussa). Schließlich kann Blausäure großtechnisch durch Dehydratisierung von Formamid hergestellt werden (BASF-Verfahren).

Bei allen vorgenannten Verfahren wird der gasförmige Reaktionsaustrag kondensiert. Das Kondensat kann Wasser enthalten. Dem Kondensat wird ein saurer Stabilisator, beispielsweise SO₂, Schwefelsäure, Phosphorsäure oder Essigsäure zugesetzt, um die autokatalytische Polymerisation von Blausäure, die in Rohrleitungen zu Verstopfungen führen kann, zu verhindern.

Blausäure wird im großen Maßstab zur Hydrocyanierung von Olefinen oder Dienen zu den entsprechenden Nitrilen eingesetzt. Die Hydrocyanierung wird üblicherweise in Gegenwart eines Nickel(0)-Katälysators, beispielsweise Tetrakis(triethylphosphit)nickel(0) oder Tetrakis(tri-p-tolylphosphit)nickel(0) durchgeführt. Dieser Nickel(0)-Katalysator ist sehr empfindlich gegenüber protischen Verbindungen wie Wasser und Säuren. So mindert die Gegenwart von Wasser in dem eingesetzten Cyanwasserstoff die Ausbeute von Adipodinitril bei der Hydrocyanierung von Butadien erheblich. Die eingesetzte Blausäure sollte daher im Wesentlichen wasser- und säurefrei sein.

US 2,571,099 beschreibt ein Verfahren zur Herstellung von Nitrilen durch Hydrocyanierung von konjugierten Diolefinen (Dienen) in Gegenwart eines Nickelcarbonyl-Katalysators. Bei dem Verfahren werden die höchsten Ausbeuten dann erzielt, wenn die eingesetzte Blausäure zumindest teilweise getrocknet wird. Im Wesentlichen wasserfreie Blausäure wird gemäß dieser Schrift durch Behandlung der wasserhaltigen Blausäure mit einem Dehydratisierungsmittel erhalten. Flüchtige saure Stabilisatoren werden zumindest teilweise dadurch entfernt, dass für einige Minuten ein Stickstoffstrom durch die Blausäure geleitet wird.

DE 33 34 321 A offenbart ein Verfahren zur Gewinnung von reiner Blausäure aus Wasser enthaltendem Acetonitril, wobei wasserfreie Blausäure in einer Destillationskolonne bei einem Druck von 1-1.5 bar und einer Kopftemperatur von 28-30°C destilliert wird

Der Einsatz von Dehydratisierungsmitteln wie beispielsweise Molsieben ist aufwendig und teuer. Diese müssen mit der wasserhaltigen Blausäure in Kontakt gebracht werden, anschließend von der entwässerten Blausäure wieder abgetrennt werden und schließlich regeneriert werden.

Aufgabe der Erfindung ist es, ein einfacheres Verfahren zur Entwässerung von Blausäure bereitzustellen.

Gelöst wird die Aufgabe durch ein Verfahren zur Entwässerung von Blausäure durch Destillation, das dadurch gekennzeichnet ist, dass eine Roh-Blausäure enthaltend 50 bis 99,9 Gew.-% HCN, 0,1 bis 40 Gew.-% Wasser, 0 bis 15 Gew.-% Kohlenstoffoxide und gegebenenfalls 0,01 bis 1 Gew.-% eines nicht flüchtigen Stabilisators, bei einem Druck von 1 bar bis 2,5 bar, einer Sumpftemperatur von 100°C bis 130°C und einer Kopftemperatur von 25°C bis 54°C in Abwesenheit eines flüchtigen Stabilisators in einer Destillationskolonne destilliert wird, wobei ein Kopfabzugsstrom, enthaltend gereinigte, wasserfreie Blausäure und Kohlenstoffoxide und ein Sumpfabzugsstrom, enthaltend Wasser und gegebenenfalls den schwerflüchtigen Stabilisator, gewonnen werden.

Überraschenderweise tritt, trotz der vergleichsweise hohen Temperaturen in der Destillationskolonne und obwohl kein flüchtiger Stabilisator eingesetzt wird, während der Destillation keine Polymerisation von Blausäure auf. Dabei ist zu bedenken, dass bei der möglichen Mitverwendung von nicht flüchtigen Stabilisatoren diese im Kolonnensumpf verbleiben und daher auch nicht oberhalb der Zufuhr der Roh-Blausäure in der Destillationskolonne stabilisierend wirken können.

Durch das erfindungsgemäße Verfahren wird eine einfache Entwässerung der wasserhaltigen Roh-Blausäure durch Destillation erreicht. Auf den Zusatz von teuren Dehydratisierungsmitteln wie Molsieben kann daher verzichtet werden. Auch werden nicht flüchtige Stabilisatoren auf diese Weise ohne weiteres abgetrennt.

Geeignete nicht flüchtige Stabilisatoren, die in der zu entwässernden Roh-Blausäure enthalten sein können, sind beispielsweise Schwefelsäure, und Phosphorsäure. Diese können anstelle von flüchtigen Stabilisatoren, wie beispielsweise Schwefeldioxid, in der Roh-Blausäure enthalten sein. Es entfällt somit auch die Abtrennung von flüchtigen Stabilisatoren, beispielsweise durch Durchleiten von Inertgas durch die Blausäure vor deren Einsatz in der Hydrocyanierungsreaktion, wie in US 2,571,099 beschrieben.

Die nach dem erfindungsgemäßen Verfahren zu entwässernde Roh-Blausäure enthält 50 bis 99,9 Gew.-%, bevorzugt 70 bis 95 Gew.-% HCN, 0,1 bis 40 Gew.-%, bevorzugt 5 bis 30 Gew.-% Wasser, 0 bis 15 Gew.-%, bevorzugt 0,1 bis 10 Gew.-% Kohlenstoffoxide (CO und CO₂) und gegebenenfalls 0,01 bis 1 Gew.-% eines nicht flüchtigen Stabilisators.

Es wird eine wasserfreie Blausäure erhalten, deren Wassergehalt im Allgemeinen < 100 ppm, bevorzugt < 10 ppm beträgt.

Das Verfahren kann auch durchgeführt werden, wenn die zu entwässernde Roh-Blausäure überhaupt keinen Stabilisator (also auch keinen nicht flüchtigen Stabilisator) enthält.

Enthält die Roh-Blausäure nicht flüchtige Stabilisatoren, so sind dies vorzugsweise Phosphorsäure oder Schwefelsäure.

Das erfindungsgemäße Verfahren kann in einer üblichen Destillationskolonne durchgeführt werden. Bevorzugt sind Glockenboden- oder Packungskolonnen.

Das erfindungsgemäße Verfahren wird vorzugsweise zur Entwässerung von wasserhaltiger Roh-Blausäure, wie sie bei der thermischen Spaltung von Formamid anfällt, durchgeführt.

Es wurde weiterhin gefunden, dass die nach dem erfindungsgemäßen Verfahren erhaltene wasserfreie Blausäure auch in Abwesenheit eines Stabilisators ohne weiteres über einen längeren Zeitraum gelagert werden kann. So kann die erhaltene wasserfreie Blausäure bei einer Temperatur von 5 bis 25°C über einen Zeitraum von zwei bis zehn Tagen oder sogar länger, beispielsweise einen Zeitraum von 5 Tagen, gelagert werden, ohne dass Polymerisation der Blausäure auftritt. Dies ist insbesondere von Bedeutung, falls die entwässerte Blausäure vor ihrer Weiterverwendung in einer Hydrocyanierungsreaktion in einem Pufferbehälter zwischengelagert werden soll.

Ist ein Pufferbehälter vorgesehen, so ist vorzugsweise der Rücklaufbehälter der Kolonne, dem der kondensierte Kopfabzugsstrom zugeführt und der Rücklauf entnommen wird, als Pufferbehälter ausgebildet.
Kohlenstoffoxide, welche mit der Blausäure über Kopf übergehen, können in einer nachgeschalteten Reinigungskolonne mit einem Inertgas, üblicher Weise Stickstoff, herausgestrippt werden.

Die gewonnene wasserfreie Blausäure kann anschließend zur Hydrocyanierung von Olefinen oder Dienen zu den entsprechenden Nitrilen eingesetzt werden.

Gegenstand der Erfindung ist somit auch ein Verfahren zur Hydrocyanierung von Olefinen oder Dienen, bei dem
a) in einem ersten Schritt Roh-Blausäure enthaltend 50 bis 99,9 Gew.-% HCN, 0,1 bis 40 Gew.-% Wasser, 0 bis 15 Gew.-% Kohlenstoffoxide und gegebenenfalls 0,01 bis 1 Gew.-% eines nicht flüchtigen Stabilisators bei einem Druck von 1 bar bis 2,5 bar, einer Sumpftemperatur von 100°C bis 130°C und einer Kopftemperatur von 25°C bis 54°C in Abwesenheit eines flüchtigen Stabilisators in einer Destillationskolonne destilliert und so entwässert wird, und die als Kopfabzugsstrom erhaltene entwässerte Blausäure in Abwesenheit eines Stabilisators gegebenenfalls gelagert wird, und
b) die gereinigte, wasserfreie Blausäure in Abwesenheit eines Stabilisators mit dem Olefin oder Dien in Gegenwart eines Hydrocyanierungskatalysators umgesetzt wird.

Die Hydrocyanierung von Olefinen und Dienen wird allgemein, wie in "Applied Homogenous Catalysis with Organometallic Compounds", Band 1, VCH Weinheim, S. 465 ff. beschrieben, in Gegenwart von Katalysatoren auf Basis von Phosphin-, Phosphit- und Phosphinit-Komplexen des Nickels oder des Palladiums durchgeführt. Zur Herstellung von Adipinsäuredinitril durch Hydrocyanierung von Butadien werden vorwiegend Nickel(0)-Phosphitkatalysatoren, gegebenenfalls in Gegenwart einer Lewis-Säure wie Metallsalze oder Triphenylbor als Promotor verwendet. Die Umsetzung erfolgt dabei in der Flüssigphase in einem Lösungsmittel, beispielsweise Tetrahydrofuran, bei einer Temperatur im Bereich von 30 bis 150 °C.

Die Erfindung wird durch die nachstehenden Beispiele näher erläutert.

### Beispiele

### Beispiel 1

### Destillation von Blausäure in Gegenwart eines nicht flüchtigen Stabilisators

In einer Glochenbodenkolonne mit einem Durchmesser von 30 mm und einer Bodenzahl von 37 werden kontinuierlich 65 g/h einer Roh-Blausäure enthaltend 99,6 Gew.-% HCN, 0,2 Gew.-% Wasser und 0,2 Gew.-% Phosphorsäure als nicht flüchtiger Stabilisator zugefahren. Die Kolonne wird bei einem Druck von 1,2 bar absolut betrieben. Dabei stellt sich am Kolonnenkopf eine Temperatur von 32°C und am Kolonnensumpf eine Temperatur von 107°C ein. Der Kopfstrom aus wasserfreier Blausäure wird kontinuierlich entnommen und in einem Pufferbehälter gesammelt. Die am Kolonnenkopf entnommene Blausäure ist klar und farblos, ihr Wassergehalt wird IR-spektrometrisch gemessen und liegt unterhalb der Nachweisgrenze von 50 ppm. Der Sumpfstrom besteht aus Wasser, hochsiedender Phosphorsäure und Spuren von HCN (im ppm-Bereich).

Die Destillation konnte über Monate hinweg stabil ohne Bildung von Ablagerungen oder Verstopfungen durch polymere Blausäure betrieben werden.

### Beispiel 2

### Destillation von Blausäure ohne Stabilisator

In einer kontinuierlich betriebenen Versuchsapparatur wird HCN durch Gasphasen-Dehydratisierung von Formamid erzeugt. Nach Kondensation von nicht umgesetztem Formamid und Entfernung des Nebenproduktes Ammoniak durch saure Wäsche aus dem Reaktionsaustrag der Formamid-Dehydratisierung wird ein Roh-Blausäure-Einsatzstrom aus 24,9 kg/h HCN, 6,7 kg/h Wasser, 1,25 kg/h CO₂ und 1,75 kg/h CO erhalten, der in die HCN-Rektifikationskolonne eingespeist wird. Die HCN-Rektifikationskolonne weist einen Durchmesser von 100 mm und eine Länge von 12 m auf und enthält eine Blechpackung, entsprechend 25 theoretischen Böden. Die Einspeisung der Roh-Blausäure erfolgt auf Höhe des 12. theoretischen Bodens. Das Wasser wird am Kolonnensumpf entnommen, die Sumpftemperatur beträgt 100°C. Der Kopfabzugsstrom besteht aus wasserfreier HCN mit einem Wassergehalt < 10 ppm, CO und CO₂. Die Kopftemperatur beträgt 23°C. Der Kopfabzugsstrom wird kondensiert und in einen nachgeschalteten Hydrocyanierungsreaktor eingespeist.

## Patentansprüche

1. Verfahren zur Entwässerung von Blausäure durch Destillation, **dadurch gekennzeichnet, dass** eine Roh-Blausäure enthaltend 50 bis 99,9 Gew.-% HCN, 0,1 bis 40 Gew.-% Wasser, 0 bis 15 Gew.-% Kohlenstoffoxide und gegebenenfalls 0,01 bis 1 Gew.-% eines nicht flüchtigen Stabilisators, bei einem Druck von 1 bar bis 2,5 bar, einer Sumpftemperatur von 100°C bis 130°C und einer Kopftemperatur von 25°C bis 54°C in Abwesenheit eines flüchtigen Stabilisators in einer Destillationskolonne destilliert wird, wobei ein Kopfabzugsstrom, enthaltend gereinigte, wasserfreie Blausäure und Kohlenstoffoxide und ein Sumpfabzugsstrom, enthaltend Wasser und gegebenenfalls den schwerflüchtigen Stabilisator, gewonnen werden.

2. Verfahren nach Anspruch 1, **dadurch gekennzeichnet, dass** die Roh-Blausäure als nicht flüchtigen Stabilisator Phosphorsäure oder Schwefelsäure enthält.

3. Verfahren nach Anspruch 1, **dadurch gekennzeichnet, dass** die Roh-Blausäure keinen Stabilisator enthält.

4. Verfahren nach einem der Ansprüche 1 bis 3, **dadurch gekennzeichnet, dass** die erhaltene gereinigte, wasserfreie Blausäure einen Wassergehalt < 100 ppm aufweist,

5. Verfahren nach einem der Ansprüche 1 bis 4, **dadurch gekennzeichnet, dass** die Destillationskolonne eine Glockenboden- oder Packungskolonne ist.

6. Verfahren nach einem der Ansprüche 1 bis 5, **dadurch gekennzeichnet, dass** die Roh-Blausäure durch thermische Spaltung von Formamid erhalten wird.

7. Verfahren nach einem der Ansprüche 1 bis 6, **dadurch gekennzeichnet, dass** die in der gereinigten, wasserfreien Blausäure enthaltenen Kohlenstoffoxide in einer nachgeschalteten Reinigungskolonne mit einem Inertgas herausgestrippt werden.

8. Verfahren nach einem der Ansprüche 1 bis 7, **dadurch gekennzeichnet, dass** die wasserfreie Blausäure in Abwesenheit eines Stabilisators bei 5 bis 25°C über einen Zeitraum von 2 bis 10 Tagen gelagert wird.

9. Verfahren zur Hydrocyanierung von Olefinen oder Dienen, bei dem
a) in einem ersten Schritt Roh-Blausäure gemäß dem Verfahren nach einem der Ansprüche 1 bis 8 entwässert und in Abwesenheit eines Stabilisators gegebenenfalls gelagert wird,
b) die wasserfreie Blausäure in Abwesenheit eines Stabilisators mit dem Olefin oder Dien in Gegenwart eines Hydrocyanierungskatalysators umgesetzt wird.

## Claims

1. A process for dewatering hydrocyanic acid by distillation, which comprises distilling crude hydrocyanic acid comprising from 50 to 99.9% by weight of HCN, from 0.1 to 40% by weight of water, from 0 to 15% by weight of carbon oxides and if appropriate from 0.01 to 1% by weight of an involatile stablilizer, at a pressure of from 1 bar to 2.5 bar, a bottom temperature of from 100°C to 130°C and a top temperature of from 25°C to 54°C, in the absence of a volatile stabilizer, in a distillation column to obtain a top draw stream comprising purified, anhydrous hydrocyanic acid and carbon oxides and a bottom draw stream comprising water and, where appropriate, the involatile stabilizer.

2. The process according to claim 1, wherein the crude hydrocyanic acid comprises, as an involatile stabilizer, phosphoric acid or sulfuric acid.

3. The process according to claim 1, wherein the crude hydrocyanic acid comprises no stabilizer.

4. The process according to any of claims 1 to 3, wherein the purified, anhydrous hydrocyanic acid obtained has a water content < 100 ppm.

5. The process according to any of claims 1 to 4, wherein the distillation column is a bubble-cap tray column or column having structured packing.

6. The process according to any of claims 1 to 5, wherein the crude hydrocyanic acid is obtained by thermally cleaving formamide.

7. The process according to any of claims 1 to 6, wherein the carbon oxides present in the purified, anhydrous hydrocyanic acid are stripped out using an inert gas in a downstream purification column.

8. The process according to any of claims 1 to 7, wherein the anhydrous hydrocyanic acid is stored at from 5 to 25°C in the absence of a stabilizer over a period of from 2 to 10 days.

9. A process for hydrocyanating olefins or dienes by
a) in a first step, dewatering crude hydrocyanic acid by a process according to any of claims 1 to 8 and if appropriate storing it in the absence of a stabilizer,
b) reacting the anhydrous hydrocyanic acid in the absence of a stabilizer with the olefin or diene in the presence of a hydrocyanation catalyst.

## Revendications

1. Procédé pour la déshydratation d'acide cyanhydrique par distillation, **caractérisé en ce qu'**un acide cyanhydrique brut contenant 50 à 99,9% en poids de HCN, 0,1 à 40% en poids d'eau, 0 à 15% en poids d'oxyde de carbone et, le cas échéant, 0,01 à 1% en poids d'un stabilisateur non volatil, est distillé sous une pression de 1 bar à 2,5 bars, à une température de fond de 100°C à 130°C et une température de tête de 25°C à 54°C en l'absence d'un stabilisateur volatil dans une colonne de distillation, un courant d'extraction de tête, contenant de l'acide cyanhydrique épuré, exempt d'eau et des oxydes de carbone de même qu'un courant d'extraction de fond, contenant de l'eau et éventuellement le stabilisateur peu volatil, sont obtenus.

2. Procédé selon la revendication 1, **caractérisé en ce que** l'acide cyanhydrique brut contient de l'acide phosphorique ou de l'acide sulfurique en tant que stabilisateur non volatil.

3. Procédé selon la revendication 1, **caractérisé en ce que** l'acide cyanhydrique brut ne contient pas de stabilisateur.

4. Procédé selon l'une des revendications 1 à 3, **caractérisé en ce que** l'acide cyanhydrique épuré, exempt d'eau obtenu présente une teneur en eau < 100 ppm.

5. Procédé selon l'une des revendications 1 à 4, **caractérisé en ce que** la colonne de distillation est une colonne à fond en cloche ou de garniture.

6. Procédé selon l'une des revendications 1 à 5, **caractérisé en ce que** l'acide cyanhydrique brut est obtenu par séparation thermique de formamide.

7. Procédé selon l'une des revendications 1 à 6, **caractérisé en ce que** les oxydes de carbone contenus dans l'acide cyanhydrique épuré, exempt d'eau, sont enlevés dans une colonne d'épuration placée en aval au moyen de gaz inerte.

8. Procédé selon l'une des revendications 1 à 7, **caractérisé en ce que** l'acide cyanhydrique exempt d'eau est conservé à une température comprise entre 5 à 25°C pendant la durée de 2 à 10 jours en l'absence d'un stabilisateur.

9. Procédé pour l'hydrocyanurisation d'oléfines ou de diènes dans lequel
a) dans une première étape, de l'acide cyanhydrique brut est déshydraté selon le procédé conforme à l'une des revendications 1 à 8 et, le cas échéant, conservé en l'absence d'un stabilisateur.
b) l'acide cyanhydrique exempt d'eau est transformé en l'absence d'un stabilisateur avec l'oléfine ou le diène en présence d'un catalyseur d'hydrocyanurisation.
